# EUROPEAN PATENT APPLICATION

(11) **EP 4 700 786 A1**
(43) Date of publication of application: **25.02.2026**
(21) Application number: 24195536.8
(22) Date of filing: 21.08.2024
(51) Int. Cl.: G16H 40/63, G06T 11/20, G06T 19/00, G16H 50/30, G16H 50/50

(54) **CONTROLLING DISPLAY**

(71) Applicant: Koninklijke Philips N.V., 5656 AG Eindhoven (NL)
(72) Inventor: BULUT, Murtaza, 5656 AG Eindhoven (NL); HUIJBREGTS, Laurentia Johanna, 5656 AG Eindhoven (NL); VAN LIESHOUT, Ron Martinus Laurentius, 5656 AG Eindhoven (NL)
(74) Representative: Philips Intellectual Property & Standards

(57) **Abstract**

Proposed concepts thus aim to provide schemes, solutions, concepts, designs, methods and systems pertaining to controlling a display of data. In particular, embodiments aim to provide a method for controlling a display of data by generating one display control signal which defines the display of an avatar of a first patient including a repeating animation, and generating another display control signal which defines the display of an avatar of a second patient using another repeating animation. In order to reduce the potential confusion and distraction of multiple animations (on the same or different display units), at least one of the display control signals is adapted to synchronize the (at least) two animations to one another. In this way, the synchronized animations are less confusing and distracting to look at, allowing a clinician to focus instead on the actual data that they are indicating.

## Description

### FIELD OF THE INVENTION

This invention relates to the field of controlling a display.

### BACKGROUND OF THE INVENTION

Avatars have recently begun being used on medical displays to provide visual representations of a subject's vital signs (and/or other relevant clinical parameters) through, for example, a virtual model of the monitored subject, indicating the healthiness of their vital signs through color, shape and/or animation. In this way, an avatar can provide an overview of a subject's health state at a quick glance. An avatar typically has a set of parameter thresholds for individual clinical parameters, and the visualization of said clinical parameters typically depends on the configuration of this parameter set. For example, if the measured values exceed or fall below a threshold, an animation can represent this transition accordingly.

For example, real-time vital sign numerical data can be mapped to visual motion, e.g., animations of an avatar. In an example method, a parameter number (e.g., a heart rate) is read at predetermined intervals (e.g., every two seconds) from a corresponding module of a patient monitor. This parameter number can then be mapped to a group (e.g., an interval and/or range) according to predetermined rules. For example, if the parameter number is more than a lower bound and less than a higher bound, then the parameter number can be mapped/linked to a group defined according to said interval. For example, this group can be a 'normal heart rate' group and can thus be represented by an animation of the avatar (e.g., the heart beating with a particular speed.

However, when many animated avatars are used in the same clinical environment or even on the same screen (such as a central monitoring station), this can lead to visual distraction and discomfort, potentially even confusing the clinicians. For example, this can then lead to mental fatigue, which if continued, can lead to physical fatigue. There is therefore a need to maintain the advantages of having multiple animated patient avatars but while reducing the potential confusion, distraction, and fatigue due to their animations.

### SUMMARY OF THE INVENTION

The invention is defined by the claims.

According to examples in accordance with an aspect of the invention, there is provided a computer-implemented method for controlling a display of data.

The method comprises: generating a first display control signal for controlling a first display of data, the first display control signal defining the display of a first avatar of a first patient, the first avatar visually indicating at least one clinical parameter of the first patient using a first repeating animation; and generating a second display control signal for controlling a second display of data, the second display control signal defining the display of a second avatar of a second patient, the second avatar visually indicating at least one clinical parameter of the second patient using a second repeating animation; and adapting at least one of the first and second display control signals to synchronize the first and second repeating animations.

Proposed concepts thus aim to provide schemes, solutions, concepts, designs, methods and systems pertaining to controlling a display of data. In particular, embodiments aim to provide a method for controlling a display of data by generating one display control signal which defines the display of an avatar of a first patient including a repeating animation, and generating another display control signal which defines the display of an avatar of a second patient using another repeating animation. In order to reduce the potential confusion and distraction of multiple animations (on the same or different display units), at least one of the display control signals is adapted to synchronize the (at least) two animations to one another. In this way, the synchronized animations are less confusing and distracting to look at, allowing a clinician to focus instead on the actual data that they are indicating.

In other words, it is proposed that by synchronizing the animations of two avatars, the cognitive load of the display(s) are advantageously reduced while still providing clinicians with the same information that was contained in the asynchronous animated avatars (and in some embodiments, even increasing the information by highlighting certain animated avatars via desynchronization).

For example, this method for controlling a display of data could be utilized to control one or more bedside medical displays, a central display (e.g., for overseeing a number of subjects in a ward), a VR/AR/MR headset, a smartphone, or any other suitable display, as would be understood by the skilled person. For example, this invention can thus facilitate the response time of a clinician being reduced, thereby leading to earlier interventions and thus fewer complications. Furthermore, this can help focus a clinician's attention on the right patient's parameter(s), thereby leading to more useful interventions and thus fewer complications. In addition, by synchronizing animations, less computing power is required to calculate and display the information on a display, thereby increasing the energy-efficiency of the display of said information.

In some embodiments, synchronizing the first and second repeating animation may comprise synchronizing the start, mid and/or end points of the first and second repeating animations. These may provide particularly effective forms of synchronization, which can substantially reduce the potential confusion and distraction caused by multiple animations. For example, preferably both the start and end points may be synchronized, and even more preferably, the start, mid and end points may be synchronized.

In some embodiments, the first and second display control signals may be for controlling the same display unit simultaneously. This may be a particularly advantageous embodiment of the invention, facilitating animations of different avatars on the same display unit being synchronized to one another.

In some embodiments, the first and second display control signals may be for controlling first and second display units respectively in the same clinical environment. This may also be a particularly advantageous embodiment of the invention, facilitating animations of different avatars on different displays, but in the same clinical environment (e.g., within the same ward), to be synchronized to one another, thus still reducing the potential confusion and/or distraction from asynchronous animations.

In some embodiments, the method may further comprise generating a third display control signal for controlling a third display of data, the third display control signal defining the display of a third avatar of a third patient, and the third avatar visually indicating at least one clinical parameter of the third patient using a third repeating animation; and adapting the third display control signal to synchronize the third repeating animation to the first and second repeating animations. This may allow for at least three animated avatars to synchronize their animations.

In some embodiments, the method may further comprise, if a clinical parameter of one of the patients exceeds a predetermined upper threshold or falls below a predetermined lower threshold, adapting the corresponding display control signal to desynchronize the corresponding repeating animation for the corresponding avatar. This may allow for a particular clinical parameter (for instance, one of concern due to it leaving a normal range) of a particular patient to essentially be highlighted by desynchronizing the animation representing said parameter from all the other animations (of other avatars and potentially within the same avatar). This would thus naturally and quickly draw a clinician's attention to said clinical parameter.

In some embodiments, the method may further comprise, if an alert related to a clinical status for one of the patients is received, adapting the corresponding display control signal to desynchronize the corresponding at least one repeating animation for the corresponding avatar. This may allow for a patient's avatar in general to be highlighted by desynchronizing one or more animations for the avatar if an alert has been received for the patient, e.g., that discharge is needed or when a predictive algorithm predicts a deterioration in the patient status, such as sepsis or hemodynamic shock, etc.

In some embodiments, the method may further comprise generating a fourth display control signal for controlling a fourth display of data, the fourth display control signal defining the display of a fourth avatar of a fourth patient, and the fourth avatar visually indicating at least one clinical parameter of the fourth patient using a fourth repeating animation, and adapting the fourth display control signal to synchronize the fourth repeating animation to the first, second, and third repeating animations; and wherein if repeating animations for two or more avatars are desynchronized for the same reason, the repeating animations for the two or more avatars may be synchronized to one another. This essentially allows for patients' avatars to be grouped together, depending on whether they have a similar clinical status, and allows for avatars (or particular clinical parameters) to be highlighted while still keeping the cognitive load of the display reduced. For instance, if the respiration rates of two patients have both left a normal range, then the animations representing said abnormal respiration rates can be desynchronized compared to the animations representing the normal respiration rates of the other patients while still being synchronized to one another, such that multiple desynchronized animations do not become overly confusing and distracting.

In some embodiments, the first avatar may visually indicate at least two clinical parameters of the first patient using at least two repeating animations respectively, and wherein the start, mid, and/or end points of the at least two repeating animations are synchronized for at least one cycle and the periods of each of the at least two repeating animations are integer multiples of the shortest period. This may allow for multiple animations within the same avatar to be synchronized to one another, while not restricting them to needing exactly the same period. Rather, the periods of the animations may only be required to be integer multiples of one another, such that, for example, the animations start/end together again after a number of cycles.

In some embodiments, the second repeating animation may have a longer period than the first repeating animation, and the end points of the first and second repeating animations may be synchronized by slowing the first repeating animation so that its new period matches the period of the second repeating animation. This may be a simple and effective way to synchronize the two repeating animations.

In some embodiments, an avatar may comprise a graphical representation of a living being. This may be a particularly effective form of avatar for indicating clinical parameters.

In some embodiments, the living being may comprise a person or an animal. These may be particularly effective forms of avatar for indicating clinical parameters for people and/or animals.

According to another aspect of the invention, there is provided a computer program comprising code means for implementing the method of any herein disclosed method when said program is run on a processing system.

According to another aspect of the invention, there is provided a system for controlling a display of data. The system comprising a processor configured to: generate a first display control signal for controlling a first display of data, the first display control signal defining the display of a first avatar of a first patient, the first avatar visually indicating at least one clinical parameter of the first patient using a first repeating animation; generate a second display control signal for controlling a second display of data, the second display control signal defining the display of a second avatar of a second patient, the second avatar visually indicating at least one clinical parameter of the second patient using a second repeating animation; and adapt at least one of the first and second display control signals to synchronize the first and second repeating animations.

In some embodiments, the system may further comprise a display unit, and the first and second display control signals may be for controlling the display unit simultaneously.

In some embodiments, the system may comprise a first display unit and a second display unit in the same clinical environment, and wherein the first and second display control signals may be for controlling the first and second display units respectively

Thus, there may be proposed concepts for controlling a display of data, and this may be done based on generating two display control signal which describe two avatars comprising at least one repeating animation respectively, and then adapting at least one of the display control signals in order to synchronize the repeating animations of the two avatars.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling a display of data according to a proposed embodiment;
Fig. 2 is a flow diagram of a method for controlling a display of data according to a proposed embodiment;
Fig. 3 is a flow diagram of a method for controlling a display of data according to a proposed embodiment;
Figs. 4A-C are block diagrams of systems for controlling a display of data according to various proposed embodiment respectively; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling a display of data. According to proposed concepts, a number of possible solutions may be implemented separately or jointly. That is, although these possible solutions may be described below separately, two or more of these possible solutions may be implemented in one combination or another.

Embodiments of the invention aim to provide a method for controlling a display of data. This can be achieved by generating one display control signal which defines the display of an avatar of a first patient including a repeating animation, and generating another display control signal which defines the display of an avatar of a second patient using another repeating animation. In order to reduce the potential confusion and distraction of multiple animations (on the same or different display units), at least one of the display control signals is adapted to synchronize the (at least) two animations to one another. In this way, the synchronized animations are less confusing and distracting to look at, allowing a clinician to focus instead on the actual data that they are indicating.

In other words, it is proposed that by synchronizing the animations of two avatars, the cognitive load required to mentally process the display(s) is advantageously reduced while still providing clinicians with the same (or even more) information that was contained in the asynchronous animated avatars.

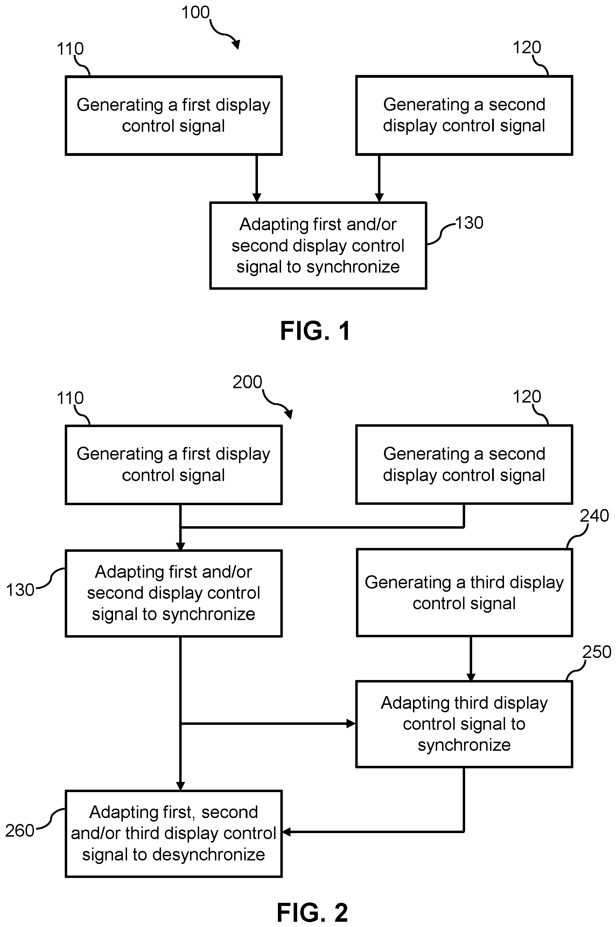

Referring now to Fig. 1, there is depicted a simplified flow diagram of a method 100 for controlling a display of data according to a proposed embodiment.

The method 100 comprises step 110 of generating a first display control signal for controlling a first display of data, the first display control signal defining the display of a first avatar of a first patient (i.e.., subject), the first avatar visually indicating at least one clinical parameter of the first patient using a first repeating animation. A display control signal can be understood as a signal capable of controlling any suitable display, e.g., a medical display, a monitor, or the display of a VR/AR/MR headset, etc., as the skilled person would understand.

Likewise, step 120 comprises generating a second display control signal for controlling a second display of data, the second display control signal defining the display of a second avatar of a second patient, the second avatar visually indicating at least one clinical parameter of the second patient using a second repeating animation. It should be noted that in some embodiments, the second patient can be the same as the first patient, such that both avatars are of the same patient.

A repeating animation can be understood as an animation that repeats after completing and can be looping (i.e., wherein its end frame matches its beginning frame (this is preferred)) or non-looping (i.e., wherein its end frame does not match its beginning frame).

In this embodiment, the first and second display control signals are for controlling the same display unit simultaneously. This is a particularly advantageous embodiment of the invention, facilitating animations of different avatars on the same display unit being synchronized to one another. For instance, this might comprise combining the first and second display control signals before outputting a joint display control signal to the single display unit. This is not essential to the working of the invention, however, as would be understood by the skilled person, and in other embodiments, the first and second display control signals can be for controlling separate display units in the same clinical environment. This is also a particularly advantageous embodiment of the invention, facilitating animations of different avatars on different displays, but in the same clinical environment (e.g., within the same ward), to be synchronized to one another, thus still reducing the potential confusion and/or distraction from asynchronous animations.

In this embodiment, an avatar comprises a graphical representation of a living being. This is a particularly effective form of avatar for indicating clinical parameters. Specifically in this embodiment, however, the living being comprises a person (i.e., the avatar comprises a graphical representation of a person). This, again, is a particularly effective form of avatar for indicating clinical parameters for people. In other embodiments, however, the living being can be an animal (e.g., for when the patient is an animal, or perhaps for children to pick for themselves).

The first and second avatars each visually indicate at least one clinical parameter of a patient using repeating animations - for example, an animation of a heart pumping quickly could be used to indicate a high heart bpm, or an animation of lungs expanding and contracting slowly could be used to indicate a low respiration rate.

In this embodiment, a clinical parameter can be a current clinical parameter (e.g., a real-time clinical parameter) or a predicted future clinical parameter - in some embodiments, all of the clinical parameters of all the patients can be current clinical parameters, but in other embodiments, they can all be predicted future clinical parameters, or a mixture of both. Accordingly, in this embodiment, a clinical parameter comprises at least one of: SpO2; heart rate; pulse rate; respiration rate; ST elevation; blood pressure; central venous pressure; end-tidal CO2; body temperature; cardiac output; cardiac index; brain activity; relaxation status; FiO2; airway pressure; tidal volume; hypotension prediction; hemodynamic instability prediction; sepsis prediction; and hypoxia prediction. These are all useful clinical parameters for a clinician to be aware of, but of course, in other embodiments, any suitable clinical parameter could be used, as would be understood by the skilled person.

Step 130 comprises adapting at least one of the first and second display control signals to synchronize the first and second repeating animations. For example, if only the first display control signal is adapted, it can be adapted to synchronize the first repeating animation with the second repeating animation (which is not changed). In another example, both the first and second display signals can be synchronized, for example, to change both the first and the second repeating animations to ensure that they are synchronized with one another. Synchronizing two animations can be understood as coordinating their temporal characteristics such that they are aligned with one another (e.g., starting or ending at the same time and/or sharing the same frame rate and/or sharing the same rate of change through the animations). Among other things, and as would be understood by the skilled person, it can involve adjusting their timing functions, such as easing or acceleration and when it begins, to create a balanced interaction where neither animation disrupts the flow of the other.

For instance, in this embodiment, synchronizing the first and second repeating animation comprises synchronizing the start, mid and/or end points of the first and second repeating animations. These provide particularly effective forms of synchronization, which can substantially reduce the potential confusion and distraction caused by multiple animations. For example, preferably both the start and end points are synchronized, and even more preferably, the start, mid and end points are synchronized. Yet even further preferably, not only are the start, mid and end points all synchronized (i.e., in alignment) but the rates of change between these points are also synchronized/matched/aligned between the animations.

For a looping animation, a mid point should be understood as the point of the animation at which it begins to move back towards the beginning state. For instance, for a looping animation that cycles back and forth between a first state and a second state (e.g., lungs that cycle between being maximally contracted (first state) and maximally expanded (second state)), the start point and the end point would both be the first state, and the mid point would be the second state. For a non-looping repeating animation, a mid point should instead be understood as the central frame of the animation (i.e., the frame that is numerically in the middle of the animation). For example, if the repeating animation is a 30-frame long non-looping animation, the mid point can be understood as the 15^{th} frame (and if the repeating animation were a 31-frame long non-looping animation, the mid point would be the 16^{th} frame (i.e., one would round up)).

As the skilled person would appreciate, there are many ways two repeating animations could be synchronized to one another, e.g., to have their start, mid, and/or end points aligned. For example, in this embodiment, the second repeating animation has a longer period (i.e., duration) than the first repeating animation, and the start (and thus also end) points of the first and second repeating animations are synchronized by slowing the first repeating animation so that its new period matches the period of the second repeating animation. This is a simple and effective way to synchronize the two repeating animations. Alternatively, however, the two repeating animations could also be synchronized by pausing the first repeating animation after completion until the second repeating animation has completed, thus aligning/synchronizing their start points.

It should also be noted that the start, mid, and end points can essentially be considered time windows that are small enough that differences in time within the window are not perceptible to the human eye. In other words, the start, mid and end points can be considered time windows smaller than 20ms, such that, for example, for the start points of two animations to be considered aligned, they need only be within 20ms of each other such that this difference cannot be noticed by the human eye.

In some embodiments, the first and/or second avatar visually indicate at least two clinical parameters of the first/second patient using at least two repeating animations respectively, and in these embodiments, adapting the first and/or second display control signals in step 130 can further be to synchronize the at least two repeating animations within the same avatar. This can be done by, for example, synchronizing the start, mid, and/or end points of the said at least two repeating animations for at least one cycle and making the periods of each of the at least two repeating animations integer multiples of the shortest period. For instance, the integer multiple could be one (so that they would have the same period) or it could be two (so that they would align their start/mid/end point every two cycles) or it could be three, etc. This would allow for multiple animations within the same avatar to be synchronized to one another, while not restricting them to needing exactly the same period. Rather the periods of the animations are only required to be integer multiples of one another, such that, for example, the animations start together again after a number of cycles (if synchronizing the start points of the animations for at least one cycle). Alternatively, in other embodiments, the periods of the animations can only be required to share a predetermined least common multiple (integer). For example, if the predetermined least common multiple is six seconds, then the first period could be two seconds, and the second period could be three seconds, such that every six seconds, the animations align again (if they started at the same time in the beginning). It should also be noted that, as above, the animations within a single avatar only need to be synchronized within 20ms of each other such that the difference cannot be noticed by the human eye.

In summary, the inventors have therefore hypothesized that when different types of motion patterns (due to multiple animated avatars) are shown simultaneously on a single screen (e.g., a central station) or multiple screens (e.g., in the clinical environment), there will be distractions due to motion-related differences among the different animated avatars. A solution is therefore to synchronize the visual changes in the avatars across different patients without losing the information given by said motion-related aspects and the connection to reference values (i.e., clinical parameters). The synchronization in this case means to align the motion-related aspects of different visual patients/avatars shown simultaneously on/in the same screen/location. Synchronization of the motion-related aspects can be important to ensure correct stratification, prioritization, and process flow.

A basic form of synchronization is to align the start and/or end points in time (phase synchronization), for animations of the avatar that can be visually observed. This will reduce the potential for confusion due to time-related dissonance between the animations while keeping the dimensional/spatial aspects of the patient avatar the same. This thus leads to a visual view with a reduced cognitive load while still providing all the necessary information. This is especially useful for stratification aspects (i.e., knowing the status of the patient).

In other words, the invention could be considered as adaptively determining how numerical (e.g., clinical parameter) data can be translated into avatar motions/animations, while taking into account the similarities and differences among the numerical data of multiple patients represented in a single view. The invention also applies, however, when the avatars are not shown on the same screen. As long as they are visualized at the same time (in the same environment), the described embodiments and benefits will still apply. For instance, a first patient avatar can be shown on a patient monitor and a second patient avatar can be shown on a mobile device, and since these are visualized at the same time by a clinician, the synchronization aspects described can still be applied. It can also be applied on individual patient monitors present in the same location (e.g., the same room).

Referring now to Fig. 2, there is depicted a flow diagram of a method 200 for controlling a display of data according to a proposed embodiment. Steps 110, 120 and 130 are substantially the same as those described in relation to the method 100 in Fig. 1.

However, method 200 further comprises step 240 of generating a third display control signal for controlling a third display of data, the third display control signal defining the display of a third avatar of a third patient, and the third avatar visually indicating at least one clinical parameter of the third patient using a third repeating animation. As would be understood by the skilled person, the third display control signal is substantially the same as the first and second display control signals have been described.

Step 250 comprises adapting the third display control signal to synchronize the third repeating animation to the first and second repeating animations (after one or both of them have been adapted, or (essentially) simultaneously). This thus allows for at least three animated avatars to synchronize their animations.

Step 260 comprises adapting either the first second and/or third display control signal to desynchronize one of the repeating animations of one of the avatars. This can be done for a number of reasons. For instance, if a (current or predicted future) clinical parameter of one of the patients exceeds a predetermined upper threshold or falls below a predetermined lower threshold, the corresponding display control signal can be adapted to desynchronize the corresponding repeating animation for the corresponding avatar. This can allow for a particular clinical parameter (for instance, one of concern due to it leaving a normal range (i.e., between the predetermined upper and lower thresholds)) of a particular patient to essentially be highlighted by desynchronizing the animation representing said parameter from all the other animations (for other avatars, and potentially, also within the same avatar). This would thus naturally and quickly draw a clinician's attention to said clinical parameter. It should be noted that in some embodiments, the predetermined upper and lower thresholds are automatically set and in others, they can be set by a user (e.g., a clinician). Even if they are automatically set, however, they can be user-adjustable.

Alternatively, or in addition, if an alert related to a clinical status for one of the patients is received, the corresponding display control signal can be adapted to desynchronize the at least one corresponding repeating animation(s) for the corresponding avatar, e.g., one or more animations of the corresponding avatar. This can allow for a patient's avatar in general to be highlighted by desynchronizing one or more animations for the avatar (from the other avatar(s)) if an alert has been received for the patient, e.g., that discharge is needed.

To be clear, an alert related to a clinical status could comprise at least one of: clinical care needed; bed assignment needed; assigned to a specific nurse; treatment needed; diagnostic needed; intervention needed; and discharge needed.

In an example, the upper and lower thresholds used for deciding whether to desynchronize the physiological parameter can be different to a predetermined 'normal range' for the physiological parameter. For instance, it is typical that for an animated avatar (alone on a patient monitor screen): threshold A and threshold B are set. If the parameter lies between A and B, an animation is shown with a certain frequency (defined as f_middle). If the parameter lies below A, an animation is shown with another frequency, f_low, with f_low < f_middle. If the parameter lies above B, an animation is shown with yet another frequency, f high, with f_high > f middle. Thresholds A and B may correspond to normal ranges and/or to alarm ranges, but not necessarily; the clinician can set them as they like. For another patient, the thresholds may be set differently (e.g. threshold C and D), but still the frequencies for the animations below, in between, and above the thresholds are f_low, f middle, and f_high respectively.

An embodiment of the invention where multiple avatars are shown on a screen and synchronization (with optional desynchronization) is utilized will now be considered. Suppose a screen displays the patients mentioned above with thresholds A and B, and thresholds C and D respectively, and a third patient with thresholds E and F. The first and second patients are clinically normal with their parameters in between thresholds A and B and thresholds C and D respectively, however, the third patient needs attention. In example 1, the third patient needs attention because their parameter is above threshold F. This automatically means that the frequency of the animation is f high, while for the first and second patient it is f middle, so this can already be seen as desynchronization. In some embodiments, it could still be "partly" synchronized if e.g. f_high is chosen to be exactly twice f_middle and the start point of f middle is synchronized with the start point of f high. Depending on urgency, it may be chosen to apply this "part" synchronization or not (i.e., "partly" synchronize them).

In a second example, the third patient needs attention but not because the parameter is above threshold F, but rather because it is approaching threshold F (but nevertheless is still between threshold E and F). For example, it may be in between threshold G and F, where threshold G lies in between threshold E and F (i.e., with threshold G representing a point close to threshold F). There is thus an extra threshold (threshold G), above which the system will trigger desynchronization. (Of course, it would be appreciated that the same principle could be applied to an extra threshold (e.g., threshold H) which represents a point close to threshold E, however, in this case, the system would trigger synchronization below threshold H). In this case, the frequency could still be f middle, so the desynchronization would therefore come from means other than the period of the animation (as described elsewhere, such as, for example, misaligning the start/end points). In some embodiments, threshold G can correspond to the upper threshold of a goal range for the parameter. This may be a stricter range than the 'normal' range and/or than the non-alarm range.

In the second example, a threshold for the parameter value itself was used. Alternatively, or additionally, thresholds for rate of change of the parameter could be used (e.g. the parameter might still in the normal/goal/non-alarm range, but is very quickly approaching a goal/alarm threshold and therefore should be desynchronized).

Also, it should be noted that desynchronization can comprise desynchronizing (de-aligning) at least one of the start, mid, and/or end points (depending on what has been synchronized, aligned) and/or merely desynchronizing the rates of change between these points between the animations (i.e., such that the start, mid and/or end points are still aligned, but the rates of change between these points differs for the desynchronized animation when compared to the synchronized animations). Preferably, however, desynchronizing comprises de-aligning at least the start and end points of the repeating animation when compared to the synchronized animations.

In other words, there can be considered three general ways to desynchronize a repeating animation with other synchronized repeating animations. One is to introduce a phase difference, so that the actual animation is exactly the same for the desynchronized animation and the synchronized animations but the start (mid) and end points of the desynchronized animation are at different times than for the synchronized animations. Another is changing the period of the desynchronized animation (i.e., either slowing it down or speeding it up) so that it no longer shares the same period with the synchronized animations and therefore repeats with its start and end points out of sync with the other synchronized animations. Finally, the period of the desynchronized animation could be kept the same as well as keeping the positions of the start (mid) and end points the same (i.e., not introducing a phase difference), but the rate of change between the start, mid, and end points could be made different (e.g., so that the synchronized animations have a constant rate of change between these points but the desynchronized animation speeds up then slows down then speeds up, for example). This final methodology, of course, is quite a subtle form of desynchronization and therefore could be used for bringing attention to less urgent matters, whereas the other forms of desynchronization could be used for more urgent matters.

In summary, prioritization is another important aspect of the present invention. For example, the risks of patient deterioration of different patients can be compared on one monitor (or between different monitors). By using input from risk scores/algorithms, the animations of a corresponding avatar can be de-synchronized to have a focus on the patient that has the highest risk or needs the fastest response. Another aspect of the invention is the process-related element - here it is important to know the risks and statuses of the patient cohort. This is especially related to workflow and resources.

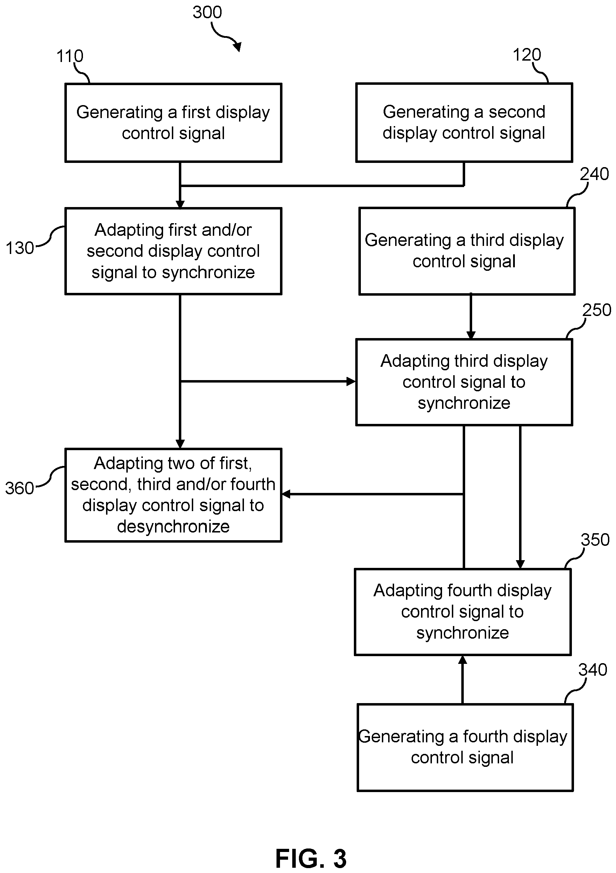

Referring now to Fig. 3, there is depicted a method 300 for controlling a display of data according to a proposed embodiment. Steps 110, 120, 130, 240 and 250 are substantially the same as those described in relation to the methods 100 and 200 in Figs. 1 and 2 respectively.

Step 340 comprises generating a fourth display control signal for controlling a fourth display of data, the fourth display control signal defining the display of a fourth avatar of a fourth patient, and the fourth avatar visually indicating at least one clinical parameter of the fourth patient using a fourth repeating animation. As would be understood by the skilled person, the fourth display control signal is substantially the same as the first, second and third display control signals have been described.

In step 350, the fourth display control signal is adapted to synchronize the fourth repeating animation to the first, second, and third repeating animations (after some or all of them have been adapted, or simultaneously). This thus allows for at least four animated avatars to synchronize their animations.

In step 360, if repeating animations for two or more avatars are desynchronized for the same reason (e.g., due to the same current or predicted future clinical parameter for two patients both leaving a predetermined range, or due to the same alert related to a clinical status for two of the patients being received), then the desynchronized repeating animations for the two or more said avatars can be synchronized to one another. This thus essentially allows for patients' avatars to be grouped/clustered together, depending on whether they have a similar clinical status, and allows for avatars (or particular clinical parameters) to be highlighted while still keeping the cognitive load of the display reduced. For instance, if the respiration rates of two patients have both left a normal range, then the animations representing said abnormal respiration rates can be desynchronized compared to the animations representing the normal respiration rates of the other patients while still being synchronized to one another, such that multiple desynchronized animations do not become overly confusing and distracting. In another example, if alerts for two patients are received indicating that they both need treatment, then one or more repeating animations for the corresponding avatars could be desynchronized from the animations of the avatars which represent patients which do not require treatment, and could instead be independently synchronized to one another (for example, they could be set to the anti-phase of the animations of the avatars which represent patients which do not require treatment). If the avatars are all being displayed on the same display unit, the position of the avatars on the screen could also be adjusted to spatially group together avatars which represent patients of similar clinical status.

In an example embodiment of the invention, how an animation of an avatar is adapted can be calculated as follows:
1. Read a *parameter number* from a patient monitor.
2. Assign a descriptive label (e.g., text) to the *parameter number.* To assign the label, use patient-specific rules. For example, a normal heart rate for patient 1 can be defined as [45 70] beats per minute, whereas for patient 2, a normal heart rate can be defined as [70 90] beats per minute. If the heart rate of patient 1 is in the normal range, then *parameter number* can be annotated as normal. Similar logic applies to the heart rate of patient 2. This means that heart rates of 50 and 80, for patient 1 and patient 2 respectively, will be mapped to the same descriptive label, e.g., *"normal".*
3. Map the descriptive label, e.g., *"normal",* to a *new number* (e.g., according to predefined rules) and then use the *new number* to drive the corresponding animation of the patient avatar (e.g., dictating the speed/period of the animation).

An alternative, different method to determine the *new_number* can be by using a *[lower_bound higher_bound]* for the *parameter_number. New_number* can be calculated as a function of *lower_bound* and *higher_bound.* For example, *new_number* could be calculated as (*lower_bound* + *higher_bound*) divided by two. In other words, as would be appreciated by the skilled person, many different methods of calculating the *new*_*number* are possible.

The mapping of measured real-time numerical data (i.e., clinical parameters) to avatar-based visualization can be represented as follows: *new*_*number* = *f(g(parameter_number))*, where *g* and *f* represent mathematical functions (rules). These two functions can be combined into a single function *F*, and we can represent the transformation *new_number* = *F(parameter_number*)*.*

In the example above, *g(parameter_number)* = *y* = *[lower_bound*, *higher_bound]*, or *g(parameter_number)* = *y* = *descriptive*_*label and f(y)* = *new*_*number.* But, for clarity, going forward, the *new_number* will be represented as *z*, and the *parameter number* will be represented as x.

Now, assuming that the *parameter number* from patient 1 is *x1*, and that *parameter number* from patient 2 is *x2,* i.e., *patient1_parameter1_number* = *x1* and *patient2_parameter1_number*= = *x2*, we will also define *parameter1* as *heart rate* and *parameter2* as *respiration rate.* An *r* can therefore be used to represent respiration rate, i.e., *r1* = *patient1_parameter2_number* and *r2* = *patient2_parameter2_number.*

Of course, heart rate and respiration rate are just two examples, and any *parameter number* (i.e., clinical parameter) that can be associated with the motion-related aspects (i.e., animations) of the patient avatar can be used.

Without any synchronization, the mapping would be as follows: *z1* = *f(g(x1)* and *z2* = *f(g(x2),* for patient 1 and patient 2 *parameter1_number* respectively. In general, there is no guarantee that *z1* = *z2.*

In this example, synchronization comprises:
(i) Selecting patients for which it will be acceptable, in the current clinical context, to have equal *new _numbers*, i.e., to make *z1* = *z2.*
(ii) Modifying the function Fto generate patient-specific transformation functions *F1* and *F2*, so that *F1(x1)* = *z1* = *F2(x2)* = *z2* = *z*. When function *F* consists of sub functions, this modification means modification of at least one sub function. For example, only g could be modified as *g1* and *g2* for patient1 and patient2 respectively, so that *g1(x1)* = *y1* = *g2(x2)* = *y2* = *y*, and therefore *f(g1(x1))* = *f(g2(x2))* = *f(y)* = *z*.
(iii) Synchronizing the phase of the modulation. In other words, for the avatars that now move with the same frequency (due to *z1* equalling *z2*), aligning the starting points of the periodic movements.

Since finding a transformation function that will generate the same result for two different inputs is a matter of relatively simple mathematics (i.e., it would be known to the skilled person), the more interesting matter is how to select patients for which it will be acceptable to have the same/synchronized visual patient representation in the clinical context. This depends on the real-time context, and what actions the system wants to suggest to the clinician at that moment. For example, several embodiments can be considered.

### Embodiment 1: Communicate that the monitored parameter(s) is/are within a normal range.

For example, at time *t1*, it may only be important to communicate that all monitoring data is within expected (*"normal*") ranges for the current patient state. In other words, all patients (patient 1, patient 2, patient 3) could be as expected, and therefore there would be no need for any immediate actions (other than regular observation).

The *"normal"* labels of *x1*, *x2*, and *x3* could therefore be mapped to a predefined z value. Alternatively, the functions could be defined without use of a descriptive label, for example, as z = *median(xl, x2, x3).* In case two monitored parameters need to be synchronized, the functions could be defined so that *z_parameter1* = *median(x1, x2, x3)* and *z_parameter2* = *median(r1*, *r2*, *r3).* As in the first example, another approach could be mapping the *x1*, *x2*, *x3* to a descriptive label and then to a *new_number.* As an extra embodiment, a special relationship between *z_parameterl* and *z_parameter2* could be desired, and the function determined accordingly. For example, in case of normal ranges, *z_parameter2* (e.g., respiration rate) could be defined as *z_parameter1*/*3* (e.g., heart rate). The relation will be determined according to the pre-specified rules.

### Embodiment 2: Emphasize (differentiate) the monitored parameters for selected patients.

At time *t2,* it may be desired to communicate to a clinician that patient 1 needs some action/attention such as bed side assistance (e.g., alarm expected to be triggered, medication change expected, bed change expected, etc.) at time *t3,* and that all other patients need an action at *t4,* where *t2<t3<t4* and *t4-t3>*10 minutes.

In this case, the avatar of patient 1 can be displayed to reflect its real values, or it can be displayed as if it were displayed on a typical bedside patient monitor. In other words, the avatar for patient 1 could be displayed without any frequency or phase synchronizations with the avatars of the other patients. The avatars of the other patients could still be displayed in a synchronized manner, however. For example, the criteria for synchronization could be derived so that all avatars of these patients are represented the same as the avatar for the patient that is expected to require some action at time *t4* - that is, from the group of patients excluding patient 1. If patient 5 is the most unstable one, and it is expected that they will require assistance at *t4,* all patients in this group (e.g., patients 2-5) can be represented with a copy of the avatar of patient 5. Of course, as the skilled person would understand, different synchronization criteria such as median, mean, quantile (similar to what has been described for the *"normal"* case), etc., can also be applied to the group of patients 2-5.

To summarize, when there is no need to bring the attention of a clinician to a specific patient, for example, when all the patient vital signs (clinical parameters) are normal, then the animations of the patients' avatars can be synchronized (e.g., in phrase, frequency, and/or visualization of the movement) with one another. When there is a need to bring the attention of a clinician to a specific patient, the animation(s) of the avatar of this patient can be selected to be de-synchronous from other patients. In the simplest case, the desynchronized animation(s) can simply be based on the real-time clinical parameter values (or similar to how an avatar is driven on typical bedside monitors, as would be understood by the skilled person). Alternatively, all patients that don't need attention could have, for example, an animation wherein the movement changes linearly in time, while for the patient that needs attention, the movement could alternate between fast and slow, yet the frequency of the total movement (up and down) and start and end points for all the avatars could still be the same - in other words, the desynchronization may only comprise desynchronization of the rate of change of the animations.

Patients can also be grouped into different clusters, consisting of one or more patients. The animations of the avatars in a particular cluster can be synchronized and determined based on a combination of patients within the cluster or based on a particular patient in said cluster.

It should also be noted that to visualize avatars that have animations with different frequencies/periods, another option can be to add 'non-motion' at the beginning or at the end. For example, if avatar 1 has an animation with period 60/60 seconds and avatar 2 has an animation that moves with period 60/80 seconds, the motion of these can be aligned so that they start or end at the same time by adding a static period to the corresponding avatar.

This invention also includes the concept of synchronizing position, i.e., clustering and repositioning patient avatars on a single display unit. In other words, in some embodiments, the definition of synchronization can be further extended to also include positioning of the patient avatars on a single screen/monitor. In other words, there can be synchronization or desynchronization not only of temporal aspects (i.e., animations), but also of spatial/coordinate aspects (i.e. positioning). Coordinate-related aspects can be determined based on different specifications. Similar patients can appear adjacent or closer to each other, and similarity can be determined based on various factors, such as: interpretation of clinical parameters, e.g., normal or abnormal; clinician preferences; responsible/assigned nurse; proximity of physical locations of the patients; and/or patient medical data, such as patient profile, reason for admission, comorbidities, ICD code, etc. In some embodiments, workflow can also determine how patient avatars are positioned and clustered, for example, patients that are scheduled to receive care within a certain time window of one another can appear close to each other; patients that follow the same protocol or are planned to follow the same protocol can appear close to one another; patients that are waiting for bed assignment can be grouped; and/or patients that are waiting to be discharged can be grouped.

In some embodiments, the definition of synchronization could also be extended to also include synchronization (i.e., matching) of the size of the avatars (or visual indications of the same physiological parameters within the avatars); a color of visual indications of the same physiological parameter within the avatars; and a line thickness of visual indications of the same physiological parameter within the avatars.

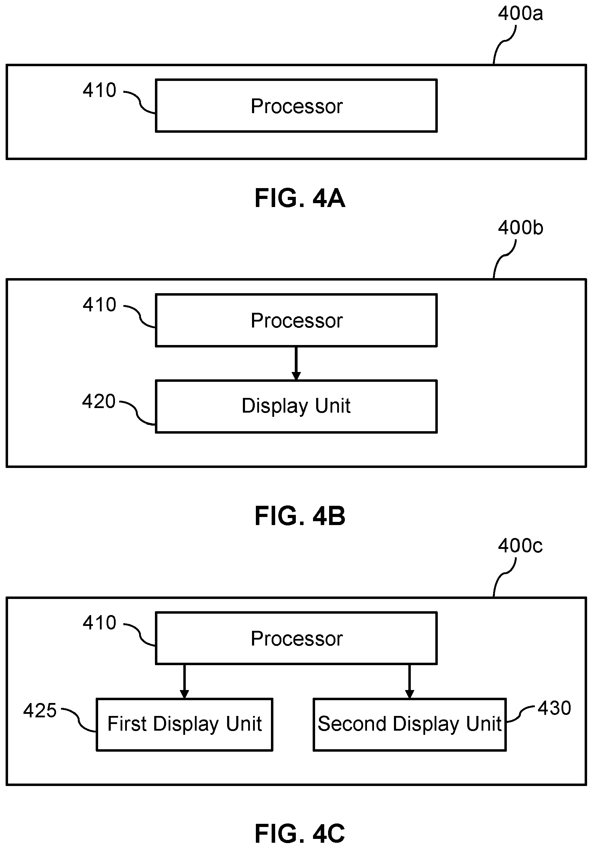

Referring now to Fig. 4A, there is depicted a block diagram of a system 400a for controlling a display of data according to a proposed embodiment. The system 400a comprises a processor 410.

The processor 410 is configured to: generate a first display control signal for controlling a first display of data, the first display control signal defining the display of a first avatar of a first patient, the first avatar visually indicating at least one clinical parameter of the first patient using a first repeating animation; and generate a second display control signal for controlling a second display of data, the second display control signal defining the display of a second avatar of a second patient, the second avatar visually indicating at least one clinical parameter of the second patient using a second repeating animation; and adapt at least one of the first and second display control signals to synchronize the first and second repeating animations. Of course, in other embodiments, the processor 410 can be configured to carry out any herein-disclosed method.

Referring now to Fig. 4B, there is depicted a block diagram of a system 400b for controlling a display of data according to another proposed embodiment. The system 400b comprises a processor 410 (substantially the same as described above) and a display unit 420. The display unit 420 can comprise any suitable display unit such as, for example, a monitor, a touch screen, a tablet screen, a smartphone screen, a VR/AR/MR headset display, etc. In this embodiment, the first and second display control signals are for controlling the display unit 420 simultaneously.

Referring now to Fig. 4C, there is depicted a block diagram of a system 400c for controlling a display of data according to another proposed embodiment. The system 400c comprises a processor 410 (substantially the same as described above), a first display unit 425, and a second display unit 430, wherein the first and second display units, 425 and 430, are in the same clinical environment. In this embodiment, the first and second display control signals are for controlling the first 425 and second 430 display units respectively.

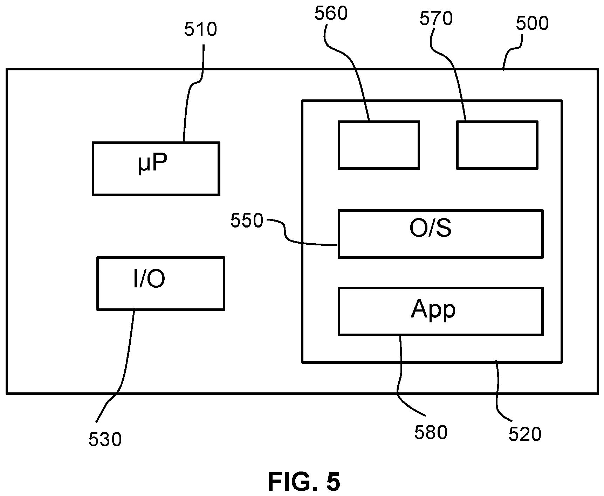

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the systems of Figs. 4A-C, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for controlling a display of data, the method comprising:
generating a first display control signal (110) for controlling a first display of data, the first display control signal defining the display of a first avatar of a first patient, the first avatar visually indicating at least one clinical parameter of the first patient using a first repeating animation; and
generating a second display control signal (120) for controlling a second display of data, the second display control signal defining the display of a second avatar of a second patient, the second avatar visually indicating at least one clinical parameter of the second patient using a second repeating animation; and
adapting at least one of the first and second display control signals (130) to synchronize the first and second repeating animations.

2. The computer-implemented method of claim 1, wherein synchronizing the first and second repeating animations comprises synchronizing the start, mid and/or end points of the first and second repeating animations.

3. The computer-implemented method of claim 1 or 2, wherein the first and second display control signals are for controlling the same display unit simultaneously.

4. The computer-implemented method of any of claims 1 or 2, wherein the first and second display control signals are for controlling first and second display units respectively in the same clinical environment.

5. The computer-implemented method of any of claims 1 to 4, wherein the method further comprises generating a third display control signal (240) for controlling a third display of data, the third display control signal defining the display of a third avatar of a third patient, and the third avatar visually indicating at least one clinical parameter of the third patient using a third repeating animation, and adapting the third display control signal (250) to synchronize the third repeating animation to the first and second repeating animations.

6. The computer-implemented method of claim 5, wherein the method further comprises, if a clinical parameter of one of the patients exceeds a predetermined upper threshold or falls below a predetermined lower threshold, adapting the corresponding display control signal (260) to desynchronize the corresponding repeating animation for the corresponding avatar.

7. The computer-implemented method of claim 5 or 6, wherein the method further comprises, if an alert related to a clinical status for one of the patients is received, adapting the corresponding display control signal (260) to desynchronize the corresponding at least one repeating animation for the corresponding avatar.

8. The computer-implemented method of any of claims 5 to 7, wherein the method further comprises generating a fourth display control signal (340) for controlling a fourth display of data, the fourth display control signal defining the display of a fourth avatar of a fourth patient, and the fourth avatar visually indicating at least one clinical parameter of the fourth patient using a fourth repeating animation, and adapting the fourth display control signal (350) to synchronize the fourth repeating animation to the first, second, and third repeating animations; and
wherein if repeating animations for two or more avatars are desynchronized for the same reason, the repeating animations for the two or more avatars are synchronized to one another.

9. The computer-implemented method of any of claims 1 to 8, wherein the first avatar visually indicates at least two clinical parameters of the first patient using at least two repeating animations respectively, and wherein the start, mid, and/or end points of the at least two repeating animations are synchronized for at least one cycle and the periods of each of the at least two repeating animations are integer multiples of the shortest period.

10. The computer-implemented method of any of claims 1 to 9, wherein the second repeating animation has a longer period than the first repeating animation, and the end points of the first and second repeating animations are synchronized by slowing the first repeating animation so that its new period matches the period of the second repeating animation.

11. The computer-implemented method of any of claims 1 to 10, wherein an avatar comprises a graphical representation of a living being.

12. A computer program product comprising computer program code means which, when executed on a computing device having a processing system, cause the processing system to perform all of the steps of the computer-implemented method according to any of claims 1 to 11.

13. A system (400a) for controlling a display of data, the system comprising:
a processor (410) configured to:
generate a first display control signal for controlling a first display of data, the first display control signal defining the display of a first avatar of a first patient, the first avatar visually indicating at least one clinical parameter of the first patient using a first repeating animation;
generate a second display control signal for controlling a second display of data, the second display control signal defining the display of a second avatar of a second patient, the second avatar visually indicating at least one clinical parameter of the second patient using a second repeating animation; and
adapt at least one of the first and second display control signals to synchronize the first and second repeating animations.

14. The system of claim 13, wherein the system (400b) further comprises a display unit (420), and the first and second display control signals are for controlling the display unit simultaneously.

15. The system of claim 13, wherein the system (400c) comprises a first display unit (425) and a second display unit (430) in the same clinical environment, and wherein the first and second display control signals are for controlling the first and second display units respectively.
